Europäisches Patentamt

(19) European Patent Office

Office européen des brevets

(11) Publication number: **0 238 334 B1**

## EUROPEAN PATENT SPECIFICATION

(45) Date of publication of patent specification :
**14.08.91 Bulletin 91/33**

(51) Int. Cl.⁵ : **A61F 13/15**

(21) Application number : **87302356.8**

(22) Date of filing : **19.03.87**

(54) **Absorbent product.**

(30) Priority : **20.03.86 JP 63340/86**
**20.03.86 JP 63341/86**

(43) Date of publication of application :
**23.09.87 Bulletin 87/39**

(45) Publication of the grant of the patent :
**14.08.91 Bulletin 91/33**

(84) Designated Contracting States :
**DE FR GB IT**

(56) References cited :
**EP-A- 0 067 916**
**EP-A- 0 137 643**
**EP-A- 0 139 484**
**DE-A- 2 532 617**
**DE-A- 2 904 634**
**GB-A- 1 480 926**
**GB-A- 2 089 214**
**US-A- 4 435 178**

(73) Proprietor : **SHISEIDO COMPANY LIMITED**
**5-5 Ginza 7-chome**
**Chuo-ku Tokyo (JP)**
Proprietor : **Mieux Products Company Ltd.**
**1349, Tokunomori**
**Oozu-shi Ehime (JP)**

(72) Inventor : **Saito, Fumiko c/o Shiseido**
**Laboratories**
**1050 Nippa-cho Kohoku-ku**
**Yokohama-shi Kanagawa (JP)**
Inventor : **Ikeda, Shinichi c/o Shiseido**
**Laboratories**
**1050 Nippa-cho Kohoku-ku**
**Yokohama-shi Kanagawa (JP)**
Inventor : **Hirano, Isao**
**c/o Shiseido Company Ltd. 7-5-5 Ginza**
**Chuo-ku Tokyo (JP)**
Inventor : **Yamagiwa, Kiyoko**
**c/o Shiseido Company Ltd. 7-5-5 Ginza**
**Chuo-ku Tokyo (JP)**
Inventor : **Kouno, Takanori**
**c/o Mieux Products Company Ltd. 1349**
**Tokunomori**
**Oozu-shi Ehime (JP)**

(74) Representative : **Coxon, Philip et al**
**Eric Potter & Clarkson St. Mary's Court St.**
**Mary's Gate**
**Nottingham NG1 1LE (GB)**

## Description

### TECHNICAL FIELD

This invention relates to a liquid-absorbent product, particularly to an absorbent product for use in sanitary napkins and diapers for babies, adults, medical purposes, and for domestic animals, or the like, for efficiently absorbing, dispersing and retaining body fluids.

### BACKGROUND ART

In conventional techniques, absorbent products used for sanitary napkins, diapers, or the like are formed in general by a top surface sheet having a liquid-permeability, a liquid-resistant bottom surface sheet sealingly joined to the front sheet at the peripheral ends thereof, and a plurality of absorbent layers comprising layers of crushed pulp, absorbent paper, polymer absorbent (sometimes referred to in the art as an superabsorbent),or the like, for absorbing and retaining body fluids such as blood, urine, mucous or other aqueous solutions or dispersions contained between the top sheet and the bottom sheet.

In an example of such an absorbent product comprising a layer of polymeric materials, body fluids absorbed therein via the liquid permeable top sheet are gelled and retained, and thus prevented from flowing out. By applying a polymer absorbent to a matrix, particularly for use in diapers, sanitary napkins, or the like which are intended to absorb relatively large volumes of liquids, the absorption capacity has been greatly increased, and as a result, absorbent products using such an absorbent layer have been made small and thin, thus, giving a relative improvement in comfort while worn. However, due to settling or bunching of the polymer powder or particles during storage or wearing, body fluids are absorbed only in an extremely limited area, and therefore, large areas of the absorbent product are not efficiently used. Also, body fluids do not disperse throughout the polymer absorbent layer, but are retained in parts of the layer. As a result, when the absorbent product is subject to a pressure, problems often occur in that the absorbed and retained fluids flow back to the top sheet, leak from the sides of the absorbent product, or even permeate the liquid resistant bottom sheet.

An absorbent product comprising a crushed pulp layer also has an excellent absorbent characteristics, but the conventional absorbent products comprising a crushed pulp layer or other absorbent layers have similar problems to those described above.

Various sanitary napkins have been proposed, but none have succeeded in preventing leakage caused by twisting, kinking, slipping, or the like of the napkins during wear.

European Specification No EP-A-0137643 discloses a liquid adsorbent product which comprises an absorbent batt (which can have an hour-glass shape) having a loosely compacted fibrous region having embossing lines formed therein and a selected densified area and a thin region forming a film on one surface thereof. The thin film can be latex. The embossed lines and the film former are employed to enhance the integrity of the batt and to enhance the tensile strength of the absorbent structure.

European Specification No EP-A-0139484 discloses a disposable sanitary product having a boat like shape and comprising a shell structure with a rim, for example a polyethylene foam. The shell contains a corrugated fibrous web which can be a polyester fibrous web. A facing is sealed to the rim of the shell to hold the web therein. The facing is a liquil permeable, generally hydrophobic web.

US Specification No US-A-4435178 discloses a disposable product which comprises an absorbent core having an upper layer of fluffed pulp and a lower layer of fluffed pulp which is embossed and printed to form coated recesses partially therethrough for accommodating and absorbing substantial discharges of material at a rate faster than otherwise possible so as to prevent leakage. A sheet of tissue is sandwiched between the fluffed pulp layers. Wadding is arranged below the lower fluffed pulp layer and above the upper fluffed pulp layers. A sheet of water impervious material overlies the upper wadding and is secured to the lower wadding. A sheet of non-woven material underlies the lower wadding and is wrapped to overlie the upper wadding and the water impervious sheet.

European Specification No EP-A-0067916 discloses a quilted diaper and sanitary napkin product in which there are provided densified regions (32,52). These regions are in linear form or are of a diamond shaped pattern. The product comprises an absorbent batt which is underline by a moisture impermeable layer. A facing layer is wrapped around the absorbent batt and the moisture impermeable layer.

GB Specification No GB-A-1480926 discloses absorbent pads which are of various hapes and have continuous linear embossed patterns. The absorbent pad comprises a fibre batt of absorbent laminated wood pulp fibres. A cover sheet of non-woven material is arranged on one side of the batt and a backing element of a thin plastics film is attached to the other side of the batt.

German Specification No DE-A-2904634 discloses an article comprising an absorbent layer having undulating linear embossed patterns, a top layer and a film arranged around the top layer and the absorbent layer. A fibrous fleece is arranged around the film and a covering layer is arranged around the fibrous layer.

## DISCLOSURE OF THE INVENTION

The present invention is intended to solve the above mentioned problems inherent in the prior art.

According to this invention there is provided an absorbent product comprising a plurality of liquid-absorbent layers including a polymer absorbent layer, a wadding layer, a crushed pulp layer, an absorbent paper sheet, and a crimped fiber layer wrapped in a liquid-permeable sheet and a U-shaped liquid-resistant sheet fixed to said liquid-permeable sheet and ends thereof, characterized in that said absorbent product is formed in a shape such that it is narrow at a middle portion of a length thereof and widened at opposite ends of the length thereof, and that at least one layer of said plurality of absorbent layers is provided with an embossed pattern formed by a plurality of offset rows of broken lines in an area away from a peripheral end of said layer, each broken line having a length of 15 to 70 mm and a space of 5 to 25 mm between the broken lines, each row of the broken lines having a space of 10 to 20 mm between adjacent rows.

The present invention may provide an absorbent product comprising liquid-absorbent layers wrapped in a liquid-permeable sheet and a liquid resistant sheet, at least one of the liquid-absorbent layers having a series of embossed patterns each formed at a certain distance in a region away from the peripheral ends thereof so that body fluids can be rapidly absorbed by and uniformly dispersed within the absorbent layers, whereby a flow back and leakage of the body fluids are eliminated and comfort during wear is improved.

The present invention may also provide an absorbent product that is comfortable when worn by designing the absorbent product in a shape such that the product is narrow at the middle portion of the length thereof and widened at each end of the length thereof.

The present invention may further provide an absorbent product comprising a liquid-resistant sheet having a U-shaped vertical sectional dimension, thereby preventing leakage of body fluids from the sides of the absorbent product.

The present invention may further provide a sanitary napkin made of the absorbent product according to the present invention in which leakage of the body fluids at the sides thereof, caused by twisting, kinking, or slipping of the napkin can be prevented by defining the napkin in the shape of a gourd having a size of 47 mm to 65 mm at the narrow portion thereof.

## BRIEF DESCRIPTION OF THE DRAWINGS

The accompanying drawings show preferable embodiments of the present invention, in which :-

Figure 1 is a perspective view of an absorbent product wherein the layers and sheets are disassembled ;

Figure 2 is a top plan view of Figure 1 ;

Figure 3 is a sectional view taken along the line A-A in Figure 2 ;

Figure 4 is a top plan view of an absorbent layer of Figure 1 ;

Figure 5 is a diagrammatical view showing the flow of body fluids around embossed patterns in an absorbent layer ;

Figure 6 is a plan view showing a dimensional relationship of a napkin in accordance with the present invention ; and

Figure 7 illustrates the relationship between a napkin and the body, when worn.

## DESCRIPTION OF THE PREFERRED EMBODIMENT

The absorbent product of the present invention comprises a plurality of liquid-absorbent layers wrapped in a liquid-permeable sheet and a liquid-resistant sheet and having a shape such that it is narrow at the middle portion of the length and widened at opposite ends of the length thereof. In the preferred embodiment, the narrowest portion and the most widest portion are connected by a natural rounded curve, as further mentioned hereinafter. The term "liquid" as used herein denotes aqueous fluids or liquids and is intended to include fluids such as saline, serum, blood, mucous, and other aqueous solutions or dispersions.

A liquid-permeable sheet and a liquid-resistant sheet in the art are generally formed in the same shape and in the same size, and are sealed at the respective peripheral ends thereof. In a preferred embodiment according to the present invention, the liquid-resistant sheet has a substantially U-shaped vertical cross section and to avoid leakage from the sides of the product, absorbent layers having substantially the same size as the bottom face of the U-shape are contained therein and sealed at the opening end of the U-shape.

According to the present invention, at least one of the absorbent layers has a plurality of embossed patterns

3

disposed at a certain distance on the inside thereof away from the peripheral ends of the layer. In the preferred embodiment, the embossed patterns are applied to all of the absorbent layers, to accelerate the permeability of body fluids permeating from the liquid-permeable top sheet through the absorbent layers to the liquid-resistant bottom sheet. In the present invention, there can be employed, alternatively or in combination, any kind of absorbent layers or sheets such as crushed pulp layer, polymer absorbent layer incorporating therein a particulated polymer absorbent, or a non-woven fabric layer subjected to needling by a water jet technique, or any other layer having an absorption capacity.

The embossed pattern of the absorbent layer according to the present invention is preferably linear, to give an efficient dispersion of fluids in the layer while maintaining the soft touch of the product although various patterns such as dot, hexagonal, and so on can be selected. Additionally, it is prefered that the patterns are disposed such that there is sufficient dispersion distance between adjacent patterns in the absorbent layer or layers. When the embossed patterns are applied to a plurality of absorbent layers, preferably the same patterns are formed at the same positions in the absorbent layers to further increase the permeability from layer to layer when piled on the liquid-resistant sheet. The expression "dispersion distance" as employed herein denotes a distance between the adjacent embossed patterns in both the up-and-down and the left-to-right directions and intended to allow a substantially uniform liquid dispersion in the absorbent layer.

Further, preferably the embossed patterns are disposed in the region away from the outer peripheral ends of an absorbent layer, to prevent the leakage of body fluids from the sides of the absorbent product when used as a sanitary napkin, diaper, or the like, and to provide a soft touch and comfortable feeling when worn.

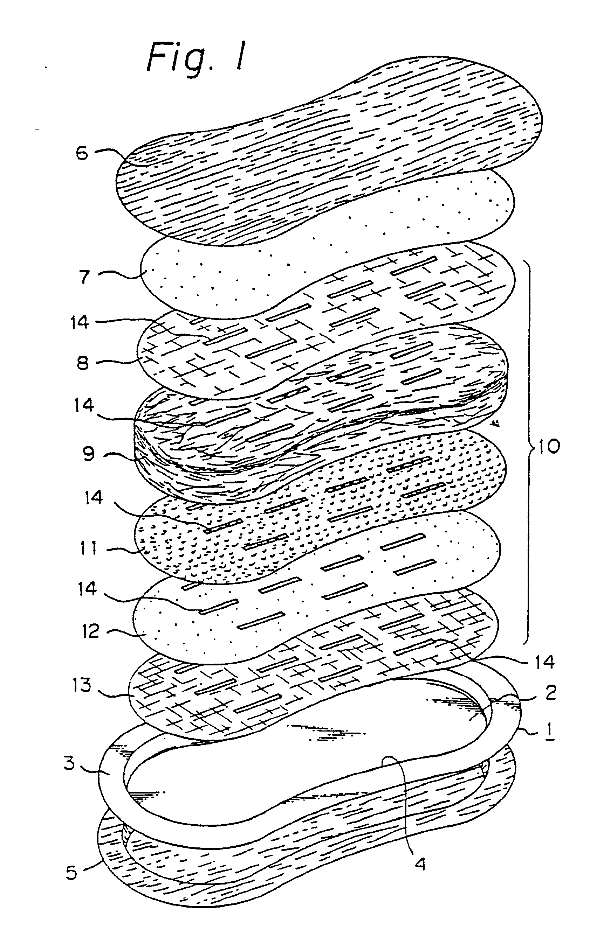

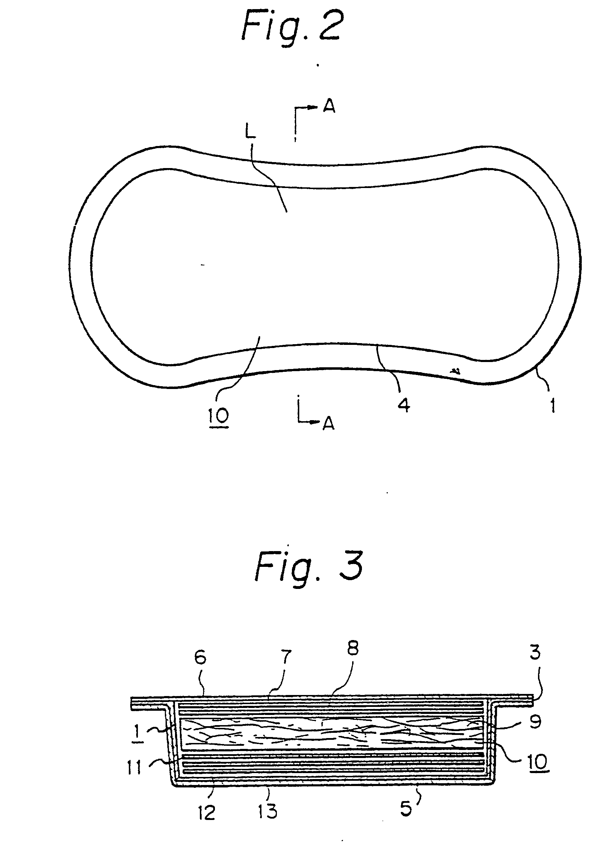

The foregoing will be better understood with reference to the drawings. The embodiments shows the absorbent product of the present invention. In Fig. 1, a liquid-resistant sheet 1 has a U-shaped vertical cross section having a container portion 2 and a flange portion 3 at an opening end and a plane bottom face having an arc-shaped narrow portion 4 at the middle of the length and an arc-shaped widened portion at the opposite ends of the length thereof. The liquid-resistant sheet may be a paper or non-woven fabric of which one or both sides are laminated with a polyethylene sheet, but is not limited thereto. On the other outer side of the liquid-resistnat sheet 1, a further bottom sheet 5 having the same shape as that of the liquid-resistant sheet 1 is provided to enhance the touch (see Fig. 1). The flange portion 3 of the liquid-resistant sheet is fixed by hot melt bonding with a liquid-permeable top sheet 6, as shown in Fig. 3. The liquid-permeable sheet is made of a non-woven fabric consisting of a polypropylene fiber and a polyethylene fiber.

Fig. 1 illustrates one embodiment according to the present invention with the layers disassembled. Referring to Fig. 1, a layer 7 made of a crimped fiber of polyester polypropylene, acetate, rayon or the like is provided underneath the liquid-permeable sheet 6. The employment of the crimped fiber layer 7 provides a better contact between the liquid-permeable sheet 6 and an absorbent paper sheet 8 by filling the space or gap between the liquid-permeable sheet 6 and the absorbent paper sheet 8, which would disturb the flow of body fluids to the absorbent paper sheet 8.

A crushed pulp layer 9 is positioned underneath the absorbent paper 8, and an absorbent wadding layer 11 is positioned underneath the crushed pulp layer 9. As the absorbent wadding layer, there can be employed a cotton or a rayon fiber per se, or the composition thereof. The crushed pulp layer 9 has an excellent absorption capacity, as is well known in the art, in that body fluids are rapidly absorbed through cellulose fibers of the pulp by a capillary action but are retained in the cellulose fibers and are not allowed to disperse. Therefore, the absorption is partial and bunched within a limited area, and is not dispersed throughout and within the pulp layer 9. In this embodiment, to allow the dispersion in the face direction of the pulp layer 9, a series of embossed patterns are formed over a defined area. The absorbent wadding layer 11 employed herein is subjected to needling by a water jet technique to accelerate the uniform dispersion and rapid absorption of the body fluids. In addition, a series of embossed patterns are formed in the same manner as in the crushed pulp layer 9 to efficiently disperse body fluids within the layer 11.

In this embodiment, an polymer absorbent layer 12 is further provided under the absorbent wadding layer 11. The employment of a polymer absorbent in the absorbent product provides a small and thin product with an increased absorbency. However, the uniform dispersion of the absorbed fluids is still unsatisfactory. To increase the dispersion rate embossed patterns are formed in the same manner as in the absorbent wadding layer 11. Note, the polymer absorbent material is not limited in this invention and can be selected from any such material employed at present in the art.

An absorbent paper sheet 13 is provided between the polymer absorbent layer 12 and the liquid-resistant sheet 1. This absorbent paper sheet 13 efficiently absorbs also body fluids leaked from the polymer absorbent layer 12 when subjected to a pressure or a large volume of liquid. The absorbent paper sheet 13 also acts as a screen for obstructing a view of body fluids through the liquid-resistant sheet 1.

The employment of the absorbent layers such as the crushed pulp layer 9, the absorbent wadding layer 11, and the polymer absorbent layer 12, can be selected according to need. In this embodiment, the absorbent paper sheet 13, polymer absorbent layer 12, the absorbent wadding layer 11, the crushed pulp layer 9, and the absorbent paper sheet 8 are all (as shown by 10 in Fig. 1) provided with a series of embossed patterns each having a linear shape 14. These respective absorbent sheets and layers are provided with the same pattern in the same position, i.e., three rows 16 composed of a series of linear embossed patterns in the longitudinal direction of the absorbent layer are provided.

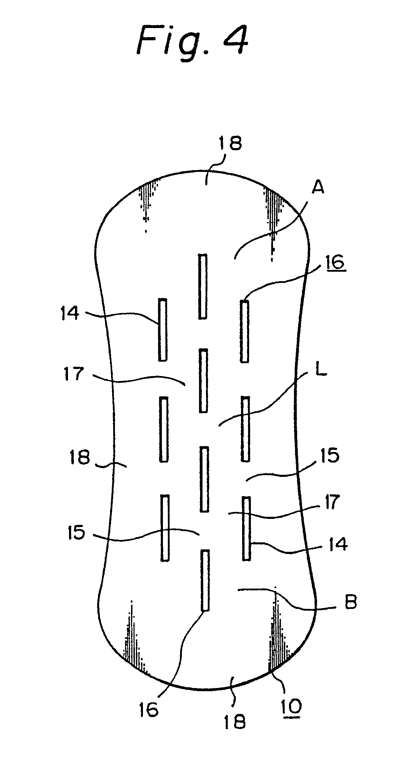

Referring now to Fig. 4, each of the embossed patterns is disposed at the dispersion distances 15 and 17 from the adjacent patterns in the longitudinal direction and transverse direction thereof, to substantially achieve a uniform dispersion within a certain area of the absorbent layer. The respective lines 16 of the adjacent linear embossed patterns are alternatively disposed. Further, the respective embossed lines are formed in an area having a certain distance 18 from the peripheral ends of an absorbent layer to define a non-dispersion area. By "non-dispersion area" as employed herein is meant an inner region along the periphery of an absorbent sheet or layer and is intended to be a region outside of the dispersion area, wherein a dispersion means is not intentionally employed. In this embodiment, the embossed pattern 14 has a width of 2 to 4 mm, and a length of 15 to 70 mm. The longitudinal dispersion distance 15 is 5 to 25 mm, and the traverse dispersion distance 17 is 10 to 20 mm. The non- dispersion distance 18 at the opposite peripheral ends is 10 to 70 mm, and at the respective opposite side areas, 10 to 25 mm.

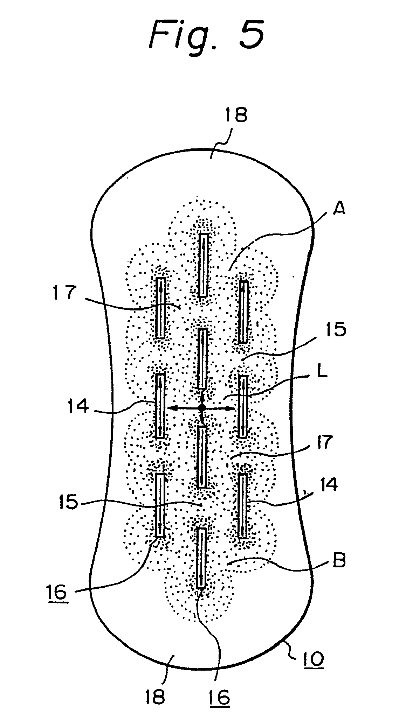

Figure 5 is a diagrammatical view explaining the flow of body fluids in a face direction of an absorbent layer via the embossed patterns, wherein the dots show the dispersion areas around the embossed patterns. Arrow lines show the directions of dispersion of the low body fluid. When body fluids are in contact with embossed patterns having a relatively high density of the materials employed as absorbent layers, with an accompanying rapid absorption by the layers, the fluids flow in the arrowed direction to the embossed patterns and expand around the patterns to form uniform dispersion areas shown by dots in the transverse directions 17 as well as the longitudinal direction 15. Even when subject to a large volume of body fluids, the body fluids can be rapidly absorbed by the layer through the embossed pattern, expand in the pattern, and flow out of the patterns to disperse around the pattern to form the dispersion areas 15 and 17 between the adjacent patterns. The employment of embossed patterns having such dispersion areas, efficiently utilizes the pulp layer 9, the polymer absorbent layer 12, and the absorbent wadding layer 11 having a relatively high absorbency. Partial bunching and partial retention of body fluids are eliminated in those absorbent layers by increasing the absorbent capacity thereof and imparting a uniform dispersion capacity. Therefore, a flow back and leakage of body fluids in the absorbent product of the present invention when used for sanitary napkins or diapers, are efficiently prevented.

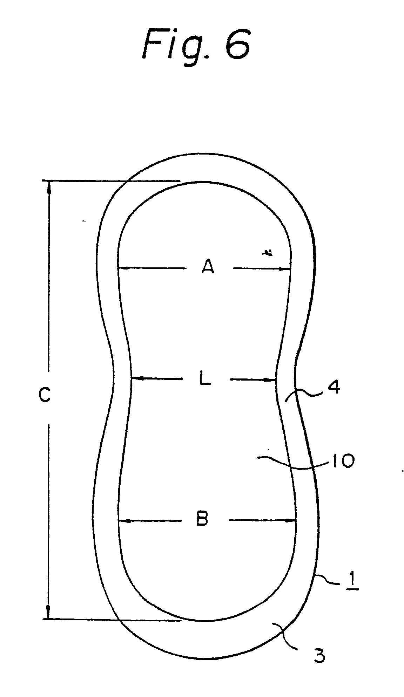

Next, a sanitary napkin made of the absorbent product as shown in Fig. 1 is explained. Figure 6 shows the dimensional relationship of the narrow portion L at the middle of the length and the widened portions at the opposite ends of the length of the absorbent layers. In Fig. 6, A and B represent the opposite widened portions, respectively. The widened portion A has a width of 54 mm - 77 mm, and the widened portion B has a width of 58 mm to 90 mm. The dimensional ratio of A : B may be in a range of 1 : 1 to 1 : 1.5. The narrow portion L has a width in the range of 47 mm to 65 mm. The length C of the absorbent layers may be 150 mm to 250 mm.

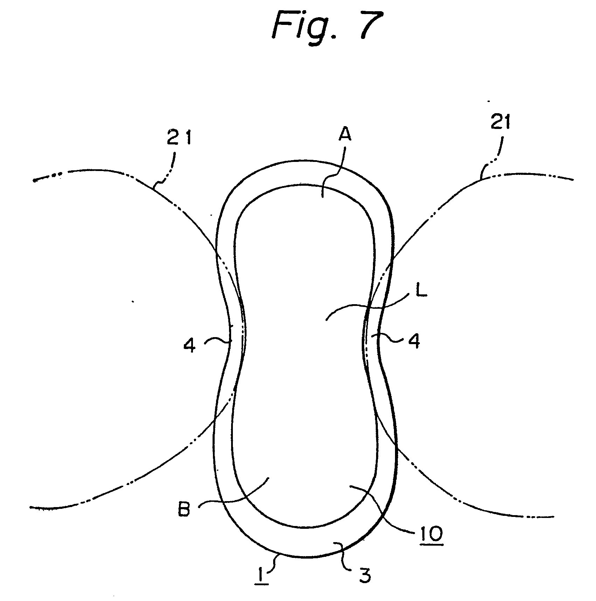

When wearing the sanitary napkin, the sides of the narrow portion fit the body 21 as shown in Fig. 7. Accordingly, there is little pressure on and consequent deformation of the side recesses 4 by the body 21. In other words, the napkin is little influenced by the movement of the body, whereby the twisting, kinking, or slipping occurring while wearing the conventional sanitary napkins is prevented.

If the dimension of the narrow portion L is too small, covering is not sufficiently executed, and accordingly, such a napkin is not useful. On the other hand, if the dimension of the narrow portion is too large, such a napkin will be easily twisted or will slip. The above-mentioned dimensions of 47 mm to 65 mm are selected on a basis of various test results, and with this size, the napkin is a comfortable fit when worn and side-linkage of the body fluids is efficiently prevented.

EXAMPLE 1

Sanitary napkins were prepared by sealing together a top sheet made of a non-woven fabric of polyethylene fiber and polypropylene fiber and a bottom sheet having a U-shaped cross section made of a laminated non-woven fabric of polyester fiber with a polyethylene sheet containing therein an absorbent paper sheet, a polymer absorbent layer, an absorbent wadding layer subjected to needling by water jet technique a crushed pulp layer, an absorbent paper sheet, and a crimped fiber layer alternately piled as shown in Fig. 1.

Each napkin was formed in the shape of a gourd, wherein each absorbent layer had an widened portion of 62 mm at the forward and 66 mm at the backward thereof, a narrow portion of the sizes as shown in Table 1, and a length of 160 mm. On the face of each absorbent layer, except for the crimped cotton layer, were formed three rows of linear embossed patterns as shown in Fig. 4. Each pattern was formed at a certain distance, had a width of 2.5 mm, and a length of 20 mm. The distance between the adjacent linear patterns in the longitudinal

5

direction was 10 mm. The distance between the adjacent pattern in the transverse direction was 12.5 mm.

The three rows of the patterns were positioned within an area 110 mm x 25 mm from the length ends and side ends respectively of the absorbent layers.

The samples were subjected to comfort, leakage, slippage, and twisting tests. Table 1 shows the results after being worn for three hours by 30 women, two days from the beginning of menstruation.

In the table, comfort is shown by the average values of the evaluation by the 30 women, and is evaluated by points, 5, 4, 3, 2, and 1 for, respectively ; "good", "fairly good", "average", "poor", and "bad". The higher points show a better comfort. Leakage is shown by percentages of the number of leakages to the number of used napkins. Slippage is shown by the average values evaluated by points, 5, 4, 3, 2, and 1 for, respectively ; "no slip at all", "no slip", "some slip", "slip", and "extreme slippage". The higher point show the napkins which had very little slip. Twisting is evaluated by the average values evaluated by points, 5, 4, 3, 2, and 1 for, respectively, "no twist at all", "no twist", "some twist", "twist", "extreme twisting". The higher values show the napkins that did not twist.

The values for each test are shown in Table 1.

Table 1

| Width of narrow portion mm | Comfort | Leakage % | Slippage | Twisting |
|---|---|---|---|---|
| 40 | 4.5 | 46 | 4.0 | 4.6 |
| 47 | 4.3 | 20 | 4.2 | 4.4 |
| 54 | 4.2 | 12 | 4.7 | 4.3 |
| 57 | 3.8 | 20 | 4.0 | 3.8 |
| 60 | 3.3 | 23 | 3.9 | 3.5 |
| 65 | 3.0 | 22 | 2.8 | 3.1 |
| 70 | 2.8 | 26 | 3.5 | 2.6 |

EXAMPLE 2

Sanitary napkins were prepared in a similar manner to Example 1 except that the narrow portion of each napkin had a width of 54 mm and the dimensional ratio of one widened portion to the other widened portion for each napkin was changed as shown in Table 2. The samples were subject to similar tests under similar conditions as in Example 1. The results are shown in Table 2.

Table 2

| Dimensional ratio | Comfort | Leakage % | Slippage | Twisting |
|---|---|---|---|---|
| 1:0.7 | 2.8 | 33 | 2.5 | 3.5 |
| 1:1 | 3.8 | 20 | 4.1 | 4.2 |
| 1:1.3 | 4.2 | 12 | 4.7 | 4.3 |
| 1:1.5 | 4.0 | 17 | 4.5 | 4.5 |
| 1:1.2 | 3.2 | 27 | 2.1 | 3.8 |

Comparative Example

For comparison, similar tests under similar conditions to those in Examples 1 and 2 were run on napkins made by similar materials and similar composition to those of Example 1 except that they have no embossed pattern in the absorbent layers and they are formed in the shape of a rectangle having a width of 70 mm and a length of an absorbent layer portion of 175 mm ; the whole length including a heat-sealed portion of 195 mm. The results for each test were as follows.

```
Table 3
Comparative    Comfort    Leakage    Slippage    Twisting
Example        2.6        26.7%      3.1         2.3
```

As seen from the test results, the napkins produced according to the present invention are far superior in each test to the Comparative Example napkin.

## Claims

1. An absorbent product comprising a plurality of liquid-absorbent layers including a polymer absorbent layer (12), a wadding layer (11), a crushed pulp layer (9), an absorbent paper sheet (8), and a crimped fiber layer (7) wrapped in a liquid-permeable sheet (6) and a U-shaped liquid-resistant sheet (1), fixed to said liquid-permeable sheet at ends thereof, said absorbent product being formed in a shape such that it is narrow at a middle portion of a length thereof and widened at opposite ends of the length thereof, and wherein at least one layer of said plurality of absorbent layers is provided with an embossed pattern (14) formed by a plurality of offset rows of broken lines in an area away from a peripheral end of said layer, each broken line having a length of 15 to 70 mm and a space (15) of 5 to 25 mm between the broken lines, each row of the broken lines having a space (L) of 10 to 20 mm between adjacent rows.

2. An absorbent product according to claim 1, in which the liquid-resistant sheet is covered by a nonwoven fabric sheet.

3. A sanitary napkin characterized by comprising the absorbent product according to claim 1 or 2.

## Patentansprüche

1. Absorbierendes Produkt mit einer Vielzahl von Flüssigkeit absorbierenden Schichten einschließlich einer absorbierenden Polymerschicht (12), einer Watteschicht (11), einer Schicht aus zerstoßener Pulpe (9), einer absorbierenden Papierschicht (8) und einer Schicht (7) aus Kräuselfasern, eingeschlagen in einer für Flüssigkeit permeablen Schicht (6) und einer U-förmigen flüssigkeitsresistenten Schicht (1), die an die für Flüssigkeit permeable Schicht an deren Enden fixiert ist, wobei das absorbierende Produkt in einer Form vorliegt, in der der Mittelabschnitt, bezogen auf seine Länge, schmal ist und die einander gegenüberliegenden Enden, bezogen auf seine Länge erweitert sind, und in dem mindestens eine Schicht der Vielzahl von absorbierenden Schichten ein ausgeprägtes Muster (14) aufweist, das von einer Mehrzahl von versetzten Reihen von unterbrochenen Zeilen in einem Bereich gebildet wird, der von einem peripheren Ende der Schicht entfernt ist, wobei jede unterbrochene Zeile eine Länge von 15 bis 70 mm aufweist und sich ein Raum (15) von 5 bis 25 mm zwischen den unterbrochenen Zeilen befindet und jede Reihe der unterbrochenen Zeilen einen Raum (L) von 10 bis 20 mm zwischen einander benachbarten Reihen aufweist.

2. Absorbierendes Produkt nach Anspruch 1, in dem die flüssigkeitsresistente Schicht von einer Schicht aus einem non-woven Stoff bedeckt ist.

3. Sanitäres Tuch, dadurch gekennzeichnet, daß es ein absorbierendes Produkt nach Ansprüchen 1 oder 2 umfaßt.

## Revendications

1. Un produit absorbant comprenant une pluralité de couches absorbantes de liquides incluant une couche absorbante polymère (12), une couche de garnissage (11), une couche de pulpe écrasée (9), une feuille de papier absorbant (8), une feuille de fibre crêpée (7) enveloppée dans une feuille perméable aux liquides (6) et une feuille en forme de U (1), résistant aux liquides et fixée aux extrémités de ladite feuille perméable aux liquides, ledit produit absorbant étant conformé de manière telle qu'il soit étroit en une partie médiane de sa longueur et élargi en ses extrémités, et dans lequel au moins une couche de cette pluralité de couches absorbantes est pourvue d'un motif en relief (14) formé d'une pluralité de rangées de lignes brisées disposées de façon décalée dans une zone espacée du bord périphérique de ladite couche, chaque segment de la ligne brisée ayant une longueur de 15 à 70 mm et les espacements (15) entre segments étant de 5 à 25 mm, un espacement (L) de 10 à 20 mm étant prévu entre chaque rangée de lignes brisées adjacentes.

2. Un produit absorbant selon la revendication 1, dans lequel la feuille résistant aux liquides est couverte d'une feuille de tissu non tissé.

3. Une serviette hygiénique caractérisée en ce qu'elle comprend le produit absorbant selon les revendications 1 ou 2

# Fig. 1

# Fig. 2

# Fig. 3

# Fig. 4

# Fig. 5

# Fig. 6

## Fig. 7